# FASCICULE DE BREVET EUROPEEN

(11) **EP 3 520 046 B1**
(45) Date de publication et mention de la délivrance du brevet: **24.12.2025**
(21) Numéro de dépôt: 16784924.9
(22) Date de dépôt: 24.10.2016
(51) Int. Cl.: G06Q 10/08, G07F 11/62, G07F 17/00, G07G 1/00

(54) **DISPOSITIF DE STOCKAGE D'ÉLÉMENTS**
LAGERUNGSVORRICHTUNG FÜR GEGENSTÄNDE
ITEM STORAGE DEVICE

(30) Priorité: 03.10.2016 FR 1659522
(43) Date de publication de la demande: 07.08.2019
(73) Titulaire: Biolog-id, 92100 Boulogne-Billancourt (FR)
(72) Inventeur: MONGRENIER, Jean-Claude, 78000 Versailles (FR)
(74) Mandataire: Lavoix
(86) Numéro de dépôt international: PCT/EP2016/075508
(87) Numéro de publication internationale: WO 2017/153009

(56) Documents cités:
- WO-A1-2010/004331
- FR-A1- 2 985 590
- KR-B1- 101 235 197
- US-A1- 2001 006 368
- US-A1- 2002 180 588
- US-A1- 2002 190 845
- US-A1- 2006 165 039
- US-A1- 2007 046 552
- US-A1- 2007 194 931
- US-A1- 2007 272 746
- US-A1- 2008 231 456
- US-A1- 2009 026 907
- US-A1- 2009 093 293
- US-A1- 2010 176 924
- US-A1- 2012 080 845
- US-A1- 2015 015 373
- US-A1- 2016 210 481
- ING ET AL: "RFID-based smart shelving storage systems Tutors", 1 January 2014 (2014-01-01), XP093003155, Retrieved from the Internet <URL:https://core.ac.uk/download/pdf/79614896.pdf> [retrieved on 20221129]
- ZARIC ANDELA ET AL: "RFID-based Smart Blood Stock System [Education Column]", IEEE ANTENNAS AND PROPAGATION MAGAZINE, IEEE SERVICE CENTER, PISCATAWAY, NJ, US, vol. 57, no. 2, 1 April 2015 (2015-04-01), pages 54 - 65, XP011581402, ISSN: 1045-9243, [retrieved on 20150515], DOI: 10.1109/MAP.2015.2420491

## Description

La présente invention concerne un dispositif de stockage d'éléments.

La présente invention se rapporte également à une installation comprenant un tel dispositif de stockage.

Les éléments sont, par exemple, des poches contenant des produits biologiques, tels que des produits sanguins (poches de sang primaire, de plasma, de plaquettes, de globules rouges...) ou des produits d'ingénieries cellulaires (cellules, souches...), ou encore des poches de médicaments, telles que des poches de chimiothérapie.

Il est connu de stocker de telles poches dans des structures réfrigérantes formées de tiroirs dans lesquels les poches sont insérées. Les poches insérées dans de telles structures comprennent généralement une étiquette identificatrice, telle qu'une étiquette RFID (de l'anglais « radio frequency identification » traduit en français par « radio identification »), dans laquelle sont mémorisées des informations relatives à la poche correspondante. En outre, un lecteur, tel qu'un lecteur RFID comprenant au moins une antenne, est disposé en vis-à-vis de l'emplacement prévu des poches de chaque tiroir afin de lire et de mettre à jour les informations contenues dans les étiquettes desdites poches.

Toutefois, des interférences sont susceptibles de se produire entre les ondes émises par l'antenne de chaque lecteur RFID, ce qui rend difficile la lecture des informations mémorisées sur les étiquettes identificatrice et donc le contrôle de l'état des éléments correspondants.

Les documents de l'état de la technique suivants comprennent des systèmes de stockage et/ ou de suivi des stocks utilisant la technologie RFID: US 2001/0006368 A1, WO 2010/004331 A1, US 2007/0272746 A1, FR 2 985 590 A1, US 2002/0180588 A1, US 2009/0189767 A1, US 2002/0190845 A1, US 2006/0165039 A1, "RFID-based smart shelving storage systems" (D'Alessandro A., University of Pisa, 2014), US 2015/0015373 A1, US 2008/0231456 A1, "RFID-based Smart Blood Stock System" (Warnick K.F., IEEE Antennas and Propagation Magazine, Vol.57, Issue 2, Avril 2015, p.54-65), KR101235197B, US 2010/0176924 A1, US 2007/0046552 A1, US 2009/0026907 A1, US 2007/0194931 A1, US 2016/0210481 A1, US 2012/0080845 A1 et US 2009/0093293 A1.

Il existe donc un besoin pour un dispositif de stockage d'éléments permettant de contrôler l'état desdits éléments de manière fiable.

A cet effet, l'invention a pour objet un dispositif selon la revendication 1.

Suivant des modes de réalisation particuliers, le dispositif comprend une ou plusieurs des caractéristiques des revendications 2 à 4, prises isolément ou suivant toutes les combinaisons techniquement possibles .

L'invention concerne aussi une installation comprenant :
- une enceinte comprenant un compartiment interne, et
- un dispositif tel que décrit ci-dessus, le dispositif étant disposé dans le compartiment interne de l'enceinte.

Suivant un mode de réalisation particulier, les éléments étant des conteneurs de plaquettes, l'enceinte est un agitateur de plaquettes.

D'autres caractéristiques et avantages de l'invention apparaîtront à la lecture de la description qui suit de modes de réalisation de l'invention, donnée à titre d'exemple uniquement et en référence aux dessins qui sont :
- figure 1, une représentation schématique en perspective d'une installation comprenant un dispositif de stockage,
- figure 2, une représentation schématique en perspective du dispositif de stockage de la figure 1,
- figure 3, une représentation schématique de plusieurs blocs tiroirs du dispositif de stockage de la figure 1, et
- figure 4, une représentation schématique d'un élément destiné à être stocké dans le dispositif de la figure 1.

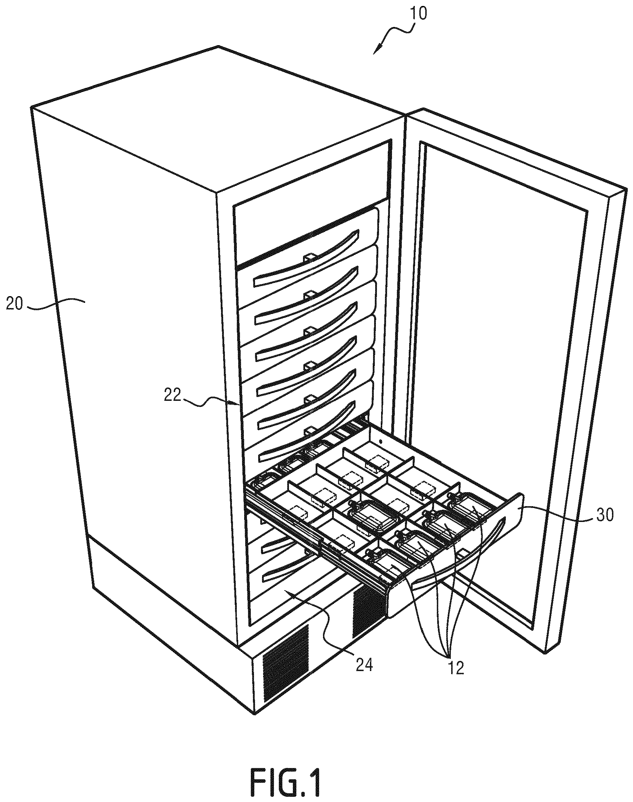

Une installation 10 de stockage d'éléments 12 est illustrée sur la figure 1.

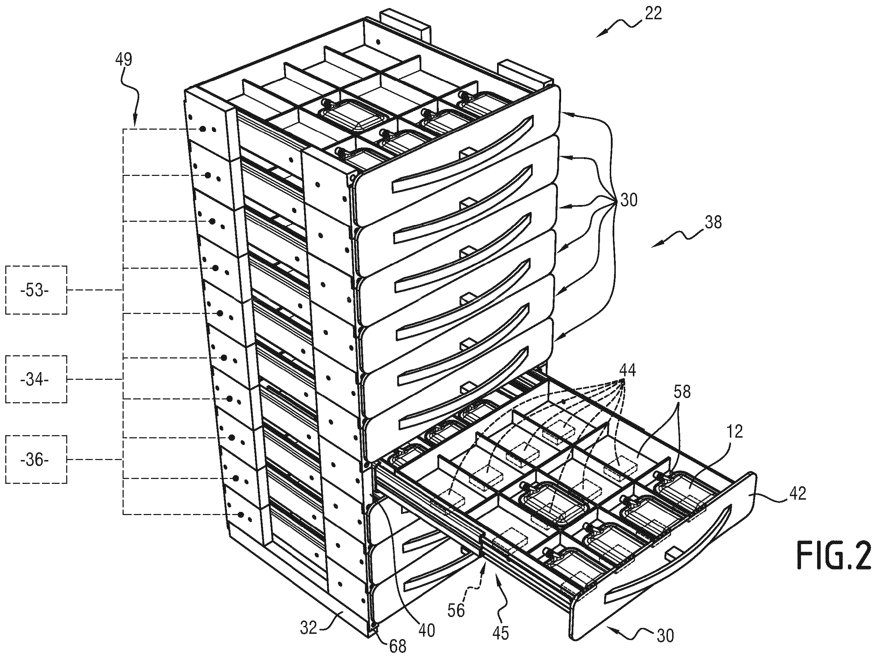

Les éléments 12 sont, par exemple, des conteneurs (visibles sur la figure 2). De manière générale, un conteneur désigne tout type de poche destiné à contenir des produits dont l'utilisation est conditionnée par des contraintes de stockage strictes.

Plus particulièrement, les éléments 12 sont, par exemple, des poches contenant des produits biologiques tels que des produits sanguins (poches de sang primaire, de plasma, de plaquettes, de globules rouges...) ou des produits d'ingénierie cellulaires (cellules humaines ou animales, notamment cellules souches humaines ou animales, produits issus de cellules humaines ou animales).

En variante, les éléments 12 sont des poches de médicaments ou des préparations thérapeutiques contenant un ou plusieurs principes actifs ou médicaments, telles que des poches de chimiothérapie contenant généralement un soluté et un ou plusieurs principes actifs de chimiothérapie.

De manière plus générale, les éléments 12 sont propres à contenir tout produit destiné à être perfusé à un humain ou à un animal.

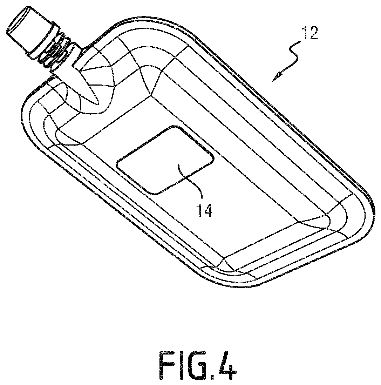

Comme visible sur la figure 4, chaque élément 12 comprend une première unité de communication sans fil 14. Chaque première unité de communication 14 est, par exemple, une étiquette, telle qu'une étiquette adhésive fixée sur une face extérieure de l'élément 12.

De manière générale, chaque première unité de communication 14 comprend au moins une antenne, une mémoire et, éventuellement, un micro-processeur.

L'antenne de chaque première unité de communication 14 est, par exemple, une antenne radiofréquence.

La mémoire de chaque première unité de communication 14 comprend des informations relatives à l'élément 12 correspondant.

De telles informations sont, par exemple : un identifiant unique de l'élément 12, la date de stockage de l'élément 12, la date de péremption de l'élément 12, la date à laquelle la première unité de communication 14 de l'élément 12 a communiqué des informations pour la dernière fois, le numéro du don relatif au contenu de l'élément 12, le code produit du contenu de l'élément 12, le groupe rhésus du contenu de l'élément 12, le phénotype sanguin du contenu de l'élément 12, l'identité du patient dont provient le contenu de l'élément 12, le nom du patient dont provient le contenu de l'élément 12, le volume du contenu de l'élément 12, le centre de don (y compris l'adresse) où a été obtenu le contenu de l'élément 12, le processus en cours sur l'élément 12 et le type d'anticoagulant du contenu de l'élément 12. Dans le cas d'une chimiothérapie, de telles informations comprennent, en outre, la date de fabrication, le type de produit, le type de véhicule, l'identité du médecin prescripteur, l'identité du pharmacien, l'identité du fabricant, la date de libération et le statut (libéré, délivré, etc).

L'installation 10 comprend une enceinte 20 et un dispositif de stockage 22.

L'enceinte 20 comprend un compartiment interne 24 de réception du dispositif 22 de stockage.

L'enceinte 20 est, par exemple, une enceinte réfrigérante, telle qu'un réfrigérateur ou un congélateur. Lorsque l'enceinte réfrigérante est un réfrigérateur, la température de l'enceinte est comprise entre 0 degrés Celsius (°C) et 5°, de préférence égale à 4°C. Lorsque l'enceinte réfrigérante est un congélateur, la température de l'enceinte est comprise entre -35°C et -196°C, de préférence égale à - 40°C.

En variante, l'enceinte 20 est un agitateur de plaquettes. L'enceinte 20 est alors de préférence intégrée dans un incubateur ayant une température, de préférence égale à 24°C.

Dans ce qui suit, il est défini des positionnements relatifs par rapport à un sens courant d'utilisation de l'enceinte 20 pour laquelle il est défini un bas, reposant généralement sur le sol, et un haut à l'opposé du bas. Ces positionnements relatifs sont notamment mis en évidence par des termes comme « en-dessous » ou « au-dessus ».

Le dispositif 22 comprend une pluralité de blocs tiroirs 30 et une base 32. Comme visible sur la figure 2, le dispositif 22 comprend, en outre, une unité de traitement 33.

Comme cela est décrit plus tard, les blocs tiroirs 30 sont empilés les uns sur les autres pour former un empilement vertical 38 de blocs tiroirs 30. Les figures 1 à 3 illustrent un exemple d'un empilement de dix blocs tiroirs 30.

Chaque bloc tiroir 30 comprend un support 40, un tiroir 42 et au moins une deuxième unité de communication 44, visible sur la figure 2.

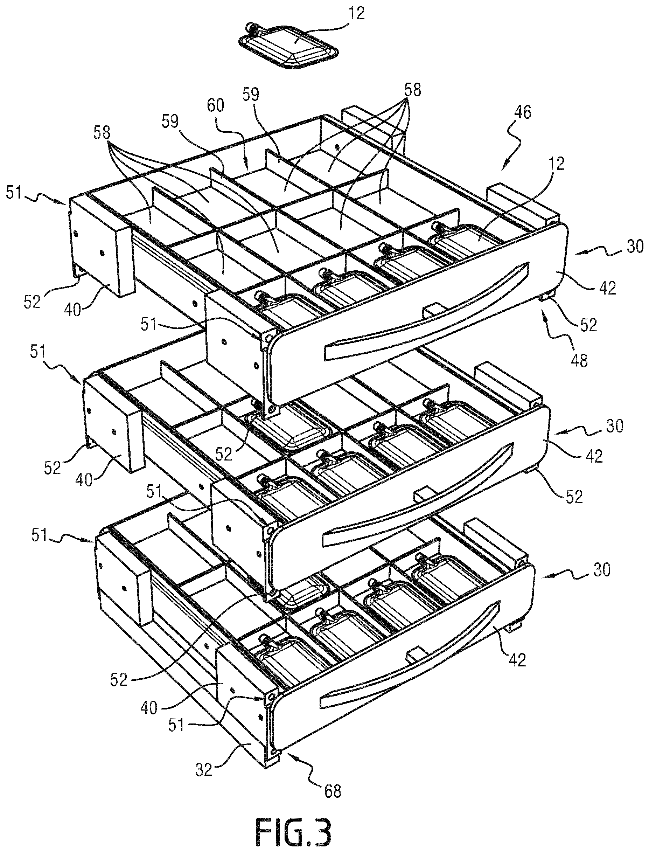

Le support 40 comprend un logement 45, une extrémité supérieure 46, une extrémité inférieure 48 (visibles sur la figure 3) et des connexions 49 (visibles sur la figure 2).

Chaque logement 45 est propre à recevoir le tiroir 42 correspondant.

L'extrémité supérieure 46 de chaque bloc tiroir 30, visible sur la figure 3, comprend au moins un premier organe d'assemblage 51. Les premiers organes d'assemblage 51 sont, par exemple, des organes d'assemblage femelles.

L'extrémité inférieure 48 de chaque bloc tiroir 30, visible sur la figure 3, comprend au moins un deuxième organe d'assemblage 52, complémentaire des premiers organes d'assemblage 51. Les deuxièmes organes d'assemblage 52 sont, par exemple, des organes d'assemblage mâles.

Dans l'exemple illustré sur la figure 3, les premiers organes d'assemblage 51 sont des fentes et les deuxièmes organes d'assemblage 52 sont des nervures complémentaires des fentes.

Ainsi, chaque bloc tiroir 30 est assemblé à au moins un autre bloc tiroir 30 de l'empilement 38 par le ou les premier(s) organe(s) d'assemblage 51 dudit bloc tiroir 30 et/ou par le ou les deuxième(s) organe(s) d'assemblage 52 dudit bloc tiroir 30.

Les connexions 49 sont, par exemple, des connexions électriques.

Dans le mode de réalisation illustré sur les figures 1 à 3, les connexions 49 de chaque bloc tiroir 30 sont connectées, d'une part, aux deuxièmes unités de communication 44 dudit bloc tiroir 30, et d'autre part, aux deuxièmes unités de communication 44 des autres blocs tiroirs 30. En outre, les connexions 49 sont connectées à l'unité de traitement 33.

Chaque tiroir 42 est positionné dans le logement 45 du support 40. Chaque tiroir 42 est propre à coulisser par rapport au support 40 correspondant.

Chaque tiroir 42 comprend un fond 56 définissant au moins un emplacement 58 de réception d'un élément 12.

Le fond 56 de chaque tiroir 42 est formé d'un matériau apte à être traversé par des ondes radioélectriques émises par la deuxième unité de communication 44 du bloc tiroir 30 dudit tiroir 42.

Le matériau du fond 56 de chaque tiroir 42 est, par exemple, du plastique.

Dans le mode de réalisation illustré sur les figures 1 à 3, le fond 56 de chaque tiroir 42 définit douze emplacements 58 de réception d'éléments 12.

Chaque emplacement 58 est, par exemple, délimité par des rebords 59 formant un casier 60.

Dans le mode de réalisation illustré sur les figures 1 à 3, chaque bloc tiroir 30 comprend autant de deuxièmes unités de communication 44 que d'emplacements 58.

Dans le mode de réalisation illustré sur les figures 1 à 3, chaque deuxième unité de communication 44 est disposée en-dessous du fond 56 du tiroir 42 en vis-à-vis de l'emplacement 58 correspondant, de sorte à permettre la communication entre ladite deuxième unité de communication 44 et la première unité de communication 14 d'un élément 12 reçu dans ledit emplacement 58.

Il est entendu par l'expression « en vis-à-vis de », le fait que chaque deuxième unité de communication 44 est disposée en face de l'espace délimité par l'emplacement 58. Autrement formulé, la projection de l'emplacement 58 dans le plan de la deuxième unité de communication 44 est confondue avec la deuxième unité de communication 44.

En variante, au moins une deuxième unité de communication 44 est disposée au-dessus de l'emplacement 58 correspondant.

Chaque deuxième unité de communication 44 est propre à communiquer, le cas échéant, avec la première unité de communication 14 de l'élément 12 reçu dans ledit emplacement 58 pour obtenir des informations relatives à l'élément 12.

Chaque deuxième unité de communication 44 est propre à émettre des ondes radiofréquences. Chaque deuxième unité de communication 44 est adaptée pour communiquer avec l'ensemble des premières unités de communication 14.

Dans l'invention, les premières unités de communication 14 sont des étiquettes RFID et les deuxièmes unités de communication 44 sont des lecteurs RFID.

Plus généralement, chaque deuxième unité de communication 44 comprend au moins une antenne, une mémoire et, éventuellement, un micro-processeur.

Le champ de rayonnement de chaque antenne couvre au moins :
- l'emplacement 58 en vis-à-vis de la deuxième unité de communication 44 de ladite antenne et,
- au moins un emplacement 58 adjacent audit emplacement 58 en vis-à-vis de la deuxième unité de communication de ladite antenne.

Plus précisément, le champ de rayonnement de chaque antenne couvre les emplacements 58 à proximité de l'antenne en fonction de l'intensité et de la forme du champ magnétique de l'antenne.

Chaque antenne comprend, en outre, deux états : un premier état dans lequel l'antenne est activée et un deuxième état dans lequel l'antenne est désactivée. Une antenne activée est une antenne propre à entrer en résonnance à une impédance prédéterminée et à une fréquence prédéterminée. L'impédance prédéterminée est, par exemple, égale à 50 Ohms (Ω) et la fréquence prédéterminée est, par exemple, égale à 13, 56 Mégahertz (MHz). Ainsi, la fréquence de résonnance d'une antenne activée est proche de la fréquence de travail de ladite antenne. Une antenne désactivée est une antenne qui n'est pas capable de résonner à la fréquence prédéterminée. La désactivation d'une antenne est effectuée, par exemple, en ouvrant la boucle de ladite antenne ou en désaccordant l'antenne de sorte que l'impédance de ladite antenne à la fréquence de résonance ait une composante réelle (ou résistive) beaucoup plus faible que la composante imaginaire (ou réactive) de ladite antenne.

Dans le mode de réalisation illustré sur les figures 1 à 3, chaque deuxième unité de communication 44 est solidaire du tiroir 42 du bloc tiroir 30 correspondant. Plus précisément, chaque deuxième unité de communication 44 est fixée en dessous du fond 56 du tiroir 42 de l'emplacement 58 correspondant.

Dans une variante, chaque deuxième unité de communication 44 est solidaire du support 40 du bloc tiroir 30 correspondant. Chaque bloc tiroir 30 comprend, en outre, un satellite. Le satellite est un boîtier qui contient la deuxième unité de communication 44. Le satellite est fixé au support 40 dudit bloc tiroir 30 directement sous le tiroir 42 dudit bloc tiroir 30. Quand le tiroir 42 est fermé, ladite deuxième unité de communication 44 est en face de l'emplacement 58 correspondant et est donc, le cas échéant, capable de communiquer avec une première unité de communication 14 positionnée dans l'emplacement 58 correspondant. Quand le tiroir 42 est ouvert, ladite deuxième unité de communication 44 n'est pas déplacée avec le tiroir 42, et par conséquent, n'est pas capable de communiquer avec une première unité de communication 14 positionnée dans l'emplacement 58 correspondant.

Optionnellement, chaque bloc tiroir 30 comprend, également, une plaque.

Chaque plaque est propre à empêcher le passage d'ondes radioélectriques émises par toutes deuxièmes unités de communication 44. Chaque plaque est, par exemple, réalisée en métal.

Chaque plaque est positionnée en-dessous du fond 56 du tiroir 42 de chaque bloc tiroir 30 et en-dessous des deuxièmes unités de communication 44 correspondant aux emplacements 58 du fond 56 du tiroir 42 dudit bloc tiroir 30. Ainsi, chaque deuxième unité de communication 44 est propre à communiquer uniquement avec les premières unités de communication 14 positionnées au-dessus de ladite deuxième unité de communication 44.

La base 32 est assemblée avec le bloc tiroir 30 le plus bas de l'empilement 38 de blocs tiroirs 30. A cet effet, la base 32 comprend une extrémité supérieure 62 comprenant au moins un troisième organe d'assemblage. Chaque troisième organe d'assemblage est identique aux premiers organes d'assemblage 51. Le ou les deuxièmes organes d'assemblage 52 du dernier bloc tiroir 30 de l'empilement 38 sont assemblés avec le ou les troisièmes organes d'assemblage de la base 32, ce qui permet de clore l'empilement 38.

L'unité de traitement 33 est connectée à chaque deuxième unité de communication 44.

L'unité de traitement 33 est propre à traiter les informations en provenance des deuxièmes unités de communication 44. En particulier, l'unité de traitement 33 est propre à commander l'activation et la désactivation de l'antenne de chaque deuxième unité de communication 44 selon une loi de commande pour déterminer si l'emplacement en vis-à-vis de la deuxième unité de communication 44 de ladite antenne est occupé par un élément 12 et, le cas échéant, les informations relatives audit élément 12.

Plus précisément, pour chaque deuxième unité de communication 44 correspondant à un emplacement 58, la loi de commande est choisie de sorte à commander en parallèle l'activation de l'antenne de ladite deuxième unité de communication 44 et la désactivation de la ou des antennes adjacentes ou en vis-à-vis de ladite antenne, ou plus généralement à proximité de ladite antenne, de sorte que l'antenne de chaque deuxième unité de communication 44 soit activée à des intervalles de temps, différents des intervalles de temps des antennes adjacentes, ou plus généralement à proximité, de ladite antenne.

La loi de commande est adaptée pour permettre à chaque deuxième unité de communication 44 de communiquer avec une éventuelle première unité de communication 14 positionnée dans l'emplacement 58 correspondant, sans interférences avec les ondes émises par les antennes des deuxièmes unités de communication 44 adjacentes à ladite deuxième unité de communication 44. Ainsi, l'antenne de chaque deuxième unité de communication 44 est activée et désactivée à intervalles de temps établis en fonction des deuxièmes unités de communication 44 adjacentes à ladite deuxième unité de communication 44.

La loi de commande dépend de la position des antennes de chaque deuxième unité de communication 44 et du champ de rayonnement desdites antennes.

Selon l'invention, il est supposé que le champ de rayonnement de chaque antenne couvre l'emplacement 58 en vis-à-vis de l'antenne et les emplacements 58 adjacents (en latéral, en haut, en bas et en diagonale) audit emplacement 58. Pour assurer le fonctionnement sans interférences d'une deuxième unité de communication donnée 44, la loi de commande commande la désactivation, pendant une durée définie, des antennes des deuxièmes unités de communication 44 en vis-à-vis des emplacements adjacents à ladite deuxième unité de communication 44 et l'activation de l'antenne de ladite deuxième unité de communication 44. La même commande est répétée à des intervalles de temps différents pour les autres deuxièmes unités de communication 44.

Dans un autre exemple, la loi de commande commande la désactivation de l'ensemble des antennes et l'activation une à une de chaque antenne à intervalles de temps différents.

Dans encore un autre exemple, la loi de commande commande l'activation d'une seule antenne par bloc tiroir 30 en choisissant des antennes qui ne sont pas en vis-à-vis les unes des autres et la désactivation de l'ensemble des autres antennes.

L'unité de traitement 33 est, en outre, propre à déterminer à quel emplacement correspond les informations recueillies par chaque antenne en fonction des informations communiquées par l'ensemble des antennes.

En effet, dans la mesure où le champ de rayonnement de chaque antenne couvre, outre l'emplacement 58 correspondant à ladite antenne, au moins un autre emplacement 58 adjacent, l'unité de traitement est configurée pour déterminer la provenance des informations recueillies par chaque antenne.

Par exemple, il est supposé que les antennes sont disposées en-dessous des tiroirs 42 et que le champ de rayonnement de chaque antenne couvre l'emplacement 58 en vis-à-vis de l'antenne (donc au-dessus de l'antenne) et l'emplacement 58 adjacent à l'antenne et situé dans le tiroir 42 en bas de l'antenne (donc en-dessous de l'antenne). L'unité de traitement 33 est propre à comparer les informations recueillies par les antennes adjacentes et à déterminer, ainsi, les informations correspondants à l'emplacement en vis-à-vis de chaque antenne. Dans cet exemple, supposons qu'une première antenne en vis-à-vis d'un premier emplacement 58 détecte un premier signal A et un deuxième signal B et qu'une deuxième antenne adjacente et directement en-dessous de la première antenne détecte le deuxième signal B et un troisième signal C. L'unité de traitement 33 détermine que le deuxième signal B détecté par chacune de la première et de la deuxième antenne provient d'une première unité de communication 44 d'un élément 12 positionné dans le premier emplacement. L'unité de traitement 33 détermine, en outre, que le premier signal A provient d'une première unité de communication 44 d'un élément 12 positionné dans l'emplacement 58 adjacent et directement au-dessus du premier emplacement 58. L'unité de traitement 33 détermine, également, que le troisième signal C provient d'une première unité de communication 44 d'un élément 12 positionné dans l'emplacement 58 adjacent et directement en-dessous du premier emplacement 58.

De manière générale, la loi de commande est choisie pour que l'unité de traitement 33 détermine de manière fiable à quel(s) emplacement(s) 58 correspondent les informations recueillies par chaque antenne.

En complément facultatif, l'unité de traitement 33 est propre à prendre en compte la force du signal, appellée RSSI (acronyme de l'anglais « return signal strength intensity », traduit en français par « intensité de puissance du signal en retour » ), émanant de chaque antenne . En pratique, plus une antenne est proche d'une première unité de communication, plus le RSSI est bon, c'est-à-dire que le RSSI est élevé. Par exemple, pour un RSSI allant de 0 à 7 : « 7 » signifie que l'antenne est au plus proche de la première unité de communication et « 0 » signifie que l'antenne ne détecte aucun signal.

Par exemple, pour un dispositif 22 comprenant six blocs tiroirs 30, cinq antennes et une première unité de communication 14 positionnée dans le troisième bloc tiroir 30, le RSSI pourrait être de {3, 6, 7, 2, 0}.

Par exemple, pour trouver où est la première unité de communication 14, l'unité de traitement 33 est propre à considérer que ladite première unité de communication 14 est entre les deux antennes dont le RSSI le plus fort. En variante, l'unité de traitement 33 est propre à déterminer ladite première unité de communication 14 en effectuant des calculs de moyenne pondérée ou de k-moyenne sur les RSSI obtenus.

Enfin, l'unité de traitement 33 est propre à déterminer l'occupation de chaque emplacement 58 et, le cas échéant, à partir desdites informations, un état de l'élément 12 positionné dans ledit emplacement 58.

Les états déterminés sont, par exemple, au nombre de deux : un état « valide » et un état « invalide ». Un élément 12 est considéré comme « valide » lorsque les informations relatives à l'élément 12 sont conformes à un cahier des charges et est considéré « invalide » dans le cas contraire.

Ainsi, l'unité de traitement 33 a une image instantanée du dispositif 22 de stockage, à savoir quel élément 12 est dans quel emplacement 58 et les informations relatives à chacun desdits éléments 12. L'unité de traitement 33 comprend également un historique des dates d'entrée et de sortie de chaque élément 12 par rapport au dispositif 22.

Optionnellement, l'unité de traitement 33 est propre à ordonner, le cas échéant, la mise à jour, par les deuxièmes unités de communication 44, des informations contenues dans les premières unités de communication 14.

Optionnellement, l'unité de traitement 33 est propre à générer une alarme en fonction de l'occupation de chaque emplacement 58 et, le cas échéant, de l'état de l'élément 12 correspondant audit emplacement 58. Par exemple, si l'unité de traitement 33 détermine qu'un même emplacement 58 comprend plus d'un élément 12, l'unité de traitement 33 génère une alarme.

Le fonctionnement du dispositif 22 intégré dans l'installation 10 va maintenant être décrit.

L'unité de traitement 33 commande l'activation et la désactivation de l'antenne de chaque deuxième unité de communication 44 selon la loi de commande. Cela permet à l'antenne de chaque deuxième unité de communication 44 de recueillir, le cas échéant, des informations relatives aux éléments 12 des emplacement 58 correspondants. L'unité de traitement 33 détermine à partir desdites informations les emplacements 58 occupés par des éléments 12 et, le cas échéant, un état des éléments 12. Le cas échéant, l'unité de traitement 33 déclenche ou non une alarme.

Ainsi, le dispositif 22 permet de contrôler de manière fiable l'état des éléments 12 stockés dans le dispositif 22, de même que le taux d'occupation des emplacements 58 des blocs tiroirs 30.

En particulier, l'activation et la désactivation des antennes en fonction de la loi de commande permet de réduire les interférences entre les signaux, ce qui améliore la lecture des informations mémorisées sur les premières unités de communication 14.

De plus, le positionnement spécifique des lecteurs en-dessous du tiroir correspondant permet d'obtenir un encombrement réduit.

Le dispositif 22 est donc un dispositif de stockage d'éléments permettant de contrôler l'état desdits éléments 12 de manière fiable sans encombrer l'espace de stockage.

En outre, il est aisé d'assembler et de désassembler les blocs tiroirs 30 du dispositif 22. Une telle modularité du dispositif 22 permet d'adapter le dispositif 22 à un grand nombre d'installations 10, en modifiant le nombre de blocs tiroirs 30 de l'empilement 38.

De plus, un empilement de blocs tiroirs est beaucoup moins lourd et encombrant qu'une pluralité de tiroirs, donc plus simple à manipuler et à installer.

En outre, d'un point de vue fabrication, des milliers de blocs tiroirs 30 identiques sont fabriquées, plutôt que des dizaines d'armoires de format différent. Cela permet d'effectuer des économies d'échelle, de simplifier la gestion du stock, ainsi que la maintenance.

Par ailleurs, le dispositif 22 est adaptable sur une installation 10 ne disposant pas préalablement de la technologie RFID.

En outre, l'encombrement réduit permet d'envisager des configurations dans lesquelles l'installation 10 contient un plus grand nombre d'éléments 12.

De plus, l'installation 10 et/ou le dispositif 22 sont aisés à fabriquer.

Enfin, dans la variante selon laquelle chaque bloc tiroir 30 comprend un satellite, le satellite est en seul bloc et est donc aisé à remplacer en cas de panne.

## Revendications

1. Dispositif (22) de stockage de conteneurs (12) de produits biologiques, chaque conteneur (12) comprenant une étiquette RFID (14) de radio-identification dans laquelle sont mémorisées des informations relatives audit conteneur (12),
le dispositif (22) comprenant :
- au moins deux blocs tiroirs (30) assemblés les uns sur les autres pour former un empilement vertical (38), chaque bloc tiroir (30) comprenant :
- un support (40) comprenant un logement (45),
- un tiroir (42) positionné dans le logement (45) du support (40) et propre à coulisser par rapport au support (40), le tiroir (42) comprenant un fond (56) définissant au moins un emplacement (58) de réception d'un conteneur (12) respectif des conteneurs (12),
- pour chaque emplacement (58), au moins un lecteur RFID (44) de radio-identification propre à émettre des ondes radiofréquences, chaque lecteur RFID (44) étant disposé en vis-à-vis de l'emplacement (58) correspondant,
chaque lecteur RFID (44) est disposé au-dessus ou en-dessous de l'emplacement (58) correspondant,
chaque lecteur RFID (44) étant adapté pour communiquer avec l'ensemble des étiquettes RFID (14), chaque lecteur RFID (44) comprenant au moins une antenne,
chaque antenne comprenant, en outre, un premier état dans lequel l'antenne est activée et un deuxième état dans lequel ladite antenne est désactivée,
chaque antenne présentant un champ de rayonnement dans le premier état, le champ de rayonnement de chaque antenne couvrant au moins :
• l'emplacement (58) en vis-à-vis du lecteur RFID (44) de ladite antenne, dit premier emplacement (58), et,
• les emplacements (58) adjacents en latéral, en haut, en bas et en diagonale audit premier emplacement, chaque antenne étant propre, le cas échéant, à recueillir des informations relatives aux conteneurs (12) des emplacements correspondants, et
- une unité de traitement de données (33) connectée à chaque lecteur RFID(44), l'unité de traitement (33) étant propre à :
- commander l'activation et la désactivation de l'antenne de chaque lecteur RFID (44) selon une loi de commande qui dépend de la position des antennes de chaque lecteur RFID (44) et du champ de rayonnement des antennes et selon laquelle l'antenne de chaque lecteur RFID (44) est activée et désactivée à des intervalles de temps établis en fonction des lecteurs RFID (44) adjacents audit lecteur RFID (44), permettant à l'unité de traitement (33) de déterminer si le premier emplacement est occupé par un conteneur (12) et, le cas échéant, les informations relatives audit conteneur (12), et
- comparer les informations recueillies par les antennes adjacentes et à déterminer, ainsi, les informations correspondantes à l'emplacement (58) en vis-à-vis de chaque antenne, et
- déterminer à quel emplacement (58) correspond les informations recueillies par chaque antenne en fonction des informations recueillies par l'ensemble des antennes.

2. Dispositif (22) selon la revendication 1, dans lequel, pour chaque lecteur (44) correspondant à un emplacement (58), la loi de commande est choisie de sorte à commander en parallèle l'activation de l'antenne de dudit lecteur (44) et la désactivation de la ou des antennes adjacentes à ladite antenne, de sorte que l'antenne de chaque lecteur (44) soit activée à des intervalles de temps différents des intervalles de temps des antennes adjacentes à ladite antenne.

3. Dispositif (22) de stockage selon la revendication 1 ou 2, dans lequel le fond (56) de chaque tiroir (42) est formé d'un matériau propre à être traversé par des ondes radioélectriques émises par des deuxièmes unités de communication (44).

4. Dispositif (22) de stockage selon l'une quelconque des revendications 1 à 3, dans lequel chaque bloc tiroir (30) comprend une plaque positionnée en-dessous du fond (56) du tiroir (42) de chaque bloc tiroir (30) et en-dessous des lecteurs (44) correspondant aux emplacements (58) du fond (56) du tiroir (42) dudit bloc tiroir (30), chaque plaque étant propre à empêcher le passage d'ondes radioélectriques émises par tous les lecteurs (44).

5. Installation (10) comprenant :
- une enceinte (20) comprenant un compartiment interne (24), et
- un dispositif (22) selon l'une quelconque des revendications 1 à 4, le dispositif (22) étant disposé dans le compartiment interne (24) de l'enceinte (20).

6. Installation (10) selon la revendication 5, dans laquelle l'enceinte (20) est un agitateur de plaquettes.

## Patentansprüche

1. Vorrichtung (22) zur Lagerung von Behältern (12) mit biologischen Produkten, jeder Behälter (12) umfassend ein RFID-Etikett (14) zur Funkidentifikation, in dem Informationen über den Behälter (12) gespeichert sind, die Vorrichtung (22) umfassend:
- mindestens zwei Schubladenblöcke (30), die übereinander montiert sind, um einen vertikalen Stapel (38) zu bilden, jeder Schubladenblock (30) umfassend:
- eine Halterung (40), umfassend ein Gehäuse (45),
- eine Schublade (42), die in der Aufnahme (45) der Halterung (40) positioniert und geeignet ist, um in Bezug auf den Träger (40) zu gleiten, die Schublade (42) umfassend einen Boden (56), der mindestens eine Position (58) zum Aufnehmen eines jeweiligen Behälters (12) der Behälter (12) definiert,
- für jede Position (58) mindestens ein RFID-Lesegerät (44) zur Funkidentifikation, das geeignet ist, um Funkwellen zu emittieren, wobei jedes RFID-Lesegerät (44) gegenüber der entsprechenden Position (58) angeordnet ist, jedes RFID-Lesegerät (44) oberhalb oder unterhalb der entsprechenden Position (58) angeordnet ist, wobei jedes RFID-Lesegerät (44) angepasst ist, um mit dem Satz von RFID-Etiketten (14) zu kommunizieren, jedes RFID-Lesegerät (44) umfassend mindestens eine Antenne, jede Antenne ferner umfassend einen ersten Zustand, in dem die Antenne aktiviert ist, und einen zweiten Zustand, in dem die Antenne deaktiviert ist, wobei jede Antenne ein Strahlungsfeld in dem ersten Zustand aufweist, wobei das Strahlungsfeld jeder Antenne mindestens Folgendes abdeckt:
• die Position (58) gegenüber dem RFID-Lesegerät (44) der Antenne, die als erste Position (58) bezeichnet wird, und,
• die Positionen (58), die an dir erste Position seitlich, oben, unten und diagonal angrenzen.
wobei jede Antenne optional geeignet ist, um Informationen über die Behälter (12) der entsprechenden Positionen zu sammeln, und
- eine Datenverarbeitungseinheit (33), die mit jedem RFID-Lesegerät(44) verbunden ist, wobei die Verarbeitungseinheit (33) zu Folgendem geeignet ist:
- Steuern der Aktivierung und Deaktivierung der Antenne von jedem RFID-Lesegerät (44) gemäß einer Steuerregel, die von der Position der Antennen von jedem RFID-Lesegerät (44) und dem Strahlungsfeld der Antennen abhängt und gemäß dem die Antenne von jedem RFID-Lesegerät (44) in Zeitintervallen aktiviert und deaktiviert wird, die abhängig von den RFID-Lesegeräten (44), die an das RFID-Lesegerät (44) angrenzen, festgelegt sind, was es der Verarbeitungseinheit (33) ermöglicht, zu bestimmen, ob die erste Position von einem Behälter (12) belegt ist, und, falls dies der Fall ist, Informationen über den Behälter (12), und
- Vergleichen der von den angrenzenden Antennen gesammelten Informationen und dadurch Bestimmen der Informationen, die der Position (58) gegenüber jeder Antenne entsprechen, und
- Bestimmen, welche Position (58) den von jeder Antenne gesammelten Informationen entspricht, basierend auf den von allen Antennen gesammelten Informationen.

2. Vorrichtung (22) nach Anspruch 1, wobei für jedes Lesegerät (44), das einer Position (58) entspricht, die Steuerregel gewählt wird, sodass die Aktivierung der Antenne des Lesegeräts (44) und die Deaktivierung der Antenne(n) angrenzend an die Antenne parallel steuert, sodass die Antenne von jedem Lesegerät (44) in Zeitintervallen aktiviert wird, die von den Zeitintervallen der Antennen angrenzend an die Antenne verschieden sind.

3. Vorrichtung (22) nach Anspruch 1 oder 2, wobei der Boden (56) jeder Schublade (42) aus einem Material gebildet ist, das geeignet ist, um von Funkwellen durchdrungen zu werden, die von zweiten Kommunikationseinheiten (44) emittiert werden.

4. Vorrichtung (22) nach einem der Ansprüche 1 bis 3, wobei jeder Schubladenblock (30) eine Platte umfasst, die unter dem Boden (56) der Schublade (42) jedes Schubladenblocks (30) und unter den Lesegeräten (44) positioniert ist, die den Positionen (58) des Bodens (56) der Schublade (42) des Schubladenblocks (30) entsprechen, wobei jede Platte geeignet ist, um den Durchgang von Funkwellen zu verhindern, die von allen Lesegeräten (44) emittiert werden.

5. Anlage (10), umfassend:
- ein Gehäuse (20), umfassend ein inneres Fach (24), und
- eine Vorrichtung (22) nach einem der Ansprüche 1 bis 4, wobei die Vorrichtung (22) in dem inneren Fach (24) des Gehäuses (20) angeordnet ist.

6. Anlage (10) nach Anspruch 5, wobei das Gehäuse (20) ein Rüttler für Blutplättchen ist.

## Claims

1. A device (22) for storing containers (12) containing biological products, each container (12) comprising a RFID tag (14) in which information relative to said container (12) is stored,
the device (22) comprising:
- at least two drawer units (30) assembled on one another to form a vertical stack (38), each drawer unit (30) comprising:
- a support (40) comprising a housing (45),
- a drawer (42) positioned in the housing (45) of the support (40) and able to slide relative to the support (40), the drawer (42) comprising a bottom (56) defining at least one location (58) for receiving a respective container (12) of the containers (12),
- for each location (58), at least one RFID reader (44) able to emit radio waves, each RFID reader (44) being arranged across from the corresponding location (58), each RFID reader (44) is arranged above or below the corresponding location (58),
each RFID reader (44) being suitable for communicating with the set of RFID tags (14), each RFID reader (44) comprising at least one antenna,
each antenna further comprising a first state in which said antenna is activated and a second state in which said antenna is deactivated,
each antenna having a radiation-zone field in the first state, the radiation-zone field of each antenna covering at least:
• the location (58) across from the RFID reader (44) of said antenna, said first location (58), and
• the locations (58) adjacent sideways, above, below and diagonally to said first location (58),
each antenna being able, if applicable, to collect information relative to the containers (12) at the corresponding locations (58),
- a data processing unit (33) connected to each RFID reader (44), the processing unit (33) being able to:
- command the activation and deactivation of the antenna of each RFID reader (44) according to a control law depending on the position of the antennas of each RFID reader (44) and of the radiation-zone field of said antennas and according to which the antenna of each RFID readers (44) is activated and deactivated at time intervals established based on the RFID readers (44) adjacent to said RFID readers (44), enabling the processing unit (33) to determine whether the first location is occupied by a container (12) and, if applicable, the information relative to said container (12), and
- to compare the information collected by the adjacent antennas and to thereby determine the information corresponding to the location (58) across from each antenna, and
- to determine the location (58) to which the information collected by each antenna corresponds based on information collected by the set of antennas.

2. The device (22) according to claim 1, wherein, for each reader (44) corresponding to a location (58), the control law is chosen so as to command, in parallel, the activation of the antenna of said reader (44) and the deactivation of the antenna(s) adjacent to said antenna, such that the antenna of each reader (44) is activated at time intervals different from the time intervals of the antennas adjacent to said antenna.

3. The storage device (22) according to claim 1 or 2, wherein the bottom (56) of each drawer (42) is made from a material able to be traversed by radio waves emitted by the second communication units (44).

4. The storage device (22) according to any one of claims 1 to 3, wherein each drawer unit (30) comprises a plate positioned below the bottom (56) of the drawer (42) of each drawer unit (30) and below the readers (44) corresponding to the locations (58) at the bottom (56) of the drawer (42) of said drawer unit (30), each plate being able to prevent the passage of radio waves emitted by all of the readers (44).

5. A facility (10), comprising:
- an enclosure (20) comprising an inner compartment (24), and
- a device (22) according to any one of claims 1 to 4, the device (22) being arranged in the inner compartment (24) of the enclosure (20).

6. The facility (10) according to claim 5, wherein the enclosure (20) is a platelet agitator.
